(19) Europäisches Patentamt
European Patent Office
Office européen des brevets

(11) Veröffentlichungsnummer: **0 206 005**
**A1**

# (12) EUROPÄISCHE PATENTANMELDUNG

(21) Anmeldenummer: **86107472.2**

(22) Anmeldetag: **02.06.86**

(51) Int. Cl.⁴: **C07D 309/06**, C07D 309/24, C07D 405/12, A01N 43/16, A01N 43/40

(30) Priorität: **15.06.85 DE 3521619**

(43) Veröffentlichungstag der Anmeldung: **30.12.86 Patentblatt 86/52**

(84) Benannte Vertragsstaaten: **CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Heinemann, Ulrich, Dr.**
**Feldstrasse 18**
**D-5653 Leichlingen 1(DE)**
Erfinder: **Hammer, Heinz, Dr.**
**Rybnikerstrasse 12**
**D-5000 Köln 80(DE)**
Erfinder: **Klug, Günter, Dr.**
**Ratherstrasse 49**
**D-4150 Krefeld(DE)**
Erfinder: **Brück, Dieter, Dr.**
**Kattowitzerstrasse 34**
**D-5000 Köln 80(DE)**
Erfinder: **Babczinski, Peter, Dr.**
**In der Lohrenbeck 11**
**D-5600 Wuppertal 1(DE)**
Erfinder: **Eue, Ludwig, Dr.**
**Paul-Klee-Strasse 36**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Santel, Hans-Joachim, Dr.**
**Grünstrasse 9a**
**D-5090 Leverkusen 1(DE)**

(54) **Pyranylalkyl-benzyl-ether.**

(57) Die Erfindung betrifft neue Pyranylalkyl-benzyl-ether der Formel (I)

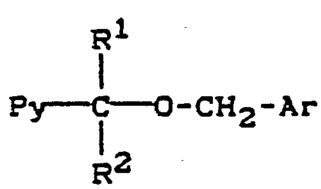

(I)

EP 0 206 005 A1

in welcher

$R^1$ und $R^2$ unabhängig voneinander jeweils für Wasserstoff oder Alkyl stehen,

Py für einen Rest

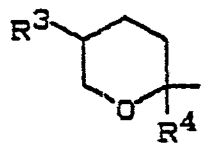

oder für einen Rest

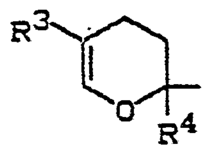

steht, wobei

$R^3$ für Wasserstoff oder Alkyl steht,

$R^4$ für Alkyl, Aralkyl, Alkoxyalkyl oder Aralkoxyalkyl steht und

Ar für jeweils gegebenenfalls substituiertes Phenyl, Naphthyl, Pyridyl odr Pyrimidyl steht,

ein Verfahren zu ihrer Herstellung, ihre Verwendung als Herbizide und neue Zwischenprodukte zur Herstellung von Pyranylalkyl-benzyl-ether der Formel (I).

## Pyranylalkyl-benzyl-ether

Die Erfindung betrifft neue Pyranylalkyl-benzylether, ein Verfahren zu ihrer Herstellung und ihre Verwendung als Herbizide.

Es ist bereits bekannt, daß bestimmte Benzylether, wie beispielsweise der (2-Methoxycyclohexyl)-(2-fluor-benzyl)-ether herbizide Eigenschaften besitzen (vgl. DE-OS 2 753 556).

Die herbizide Wirksamkeit dieser vorbekannten Verbindungen gegenüber Schadpflanzen ist jedoch ebenso wie ihre Verträglichkeit gegenüber wichtigen Kulturpflanzen nicht immer in allen Anwendungsbereichen völlig zufriedenstellend.

Es wurden neue Pyranylalkyl-benzyl-ether der allgemeinen Formel (I),

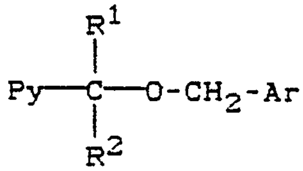

(I)

in welcher

$R^1$ und $R^2$ unabhängig voneinander jeweils für Wasserstoff oder Alkyl stehen,

Py für einen Rest

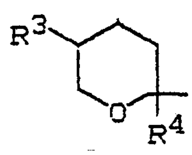

oder für einen Rest

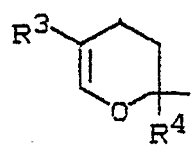

steht, wobei

$R^3$ für Wasserstoff oder Alkyl steht,

$R^4$ für Alkyl, Aralkyl, Alkoxyalkyl oder Aralkoxyalkyl steht und

Ar für jeweils gegebenenfalls substituiertes Phenyl, Naphthyl, Pyridyl oder Pyrimidyl steht,

sowie deren Salze gefunden.

Die erfindungsgemäßen Wirkstoffe der Formel (I) können als geometrische und/oder optische Isomere oder Isomerengemische auftreten. Sowohl die reinen Isomeren als auch die Isomerengemische werden erfindungsgemäß beansprucht.

Weiterhin wurde gefunden, daß man die neuen Pyranylalkyl-benzyl-ether der allgemeinen Formel -(I),

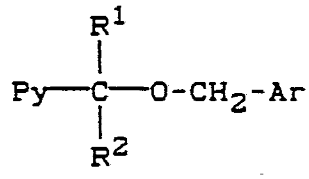

(I)

in welcher

$R^1$ und $R^2$ unabhängig voneinander jeweils für Wasserstoff oder Alkyl stehen,

Py für einen Rest

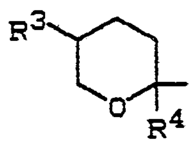

oder für einen Rest

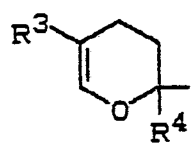

steht, wobei

$R^3$ für Wasserstoff oder Alkyl steht,

$R^4$ für Alkyl, Aralkyl, Alkoxyalkyl oder Aralkoxyalkyl steht und

Ar für jeweils gegebenenfalls substituiertes Phenyl, Naphthyl, Pyridyl oder Pyrimidyl steht,

erhält, wenn man

Pyranylalkylcarbinole der Formel (II),

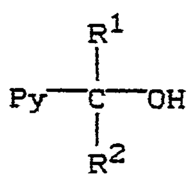

(II)

in welcher

$R^1$, $R^2$ und Py die oben angegebene Bedeutung haben,

mit Arylalkylhalogeniden der Formel (III),

$Ar-CH_2-Hal$ (III)

in welcher

Ar die oben angegebene Bedeutung hat und

Hal für Halogen steht,

gegebenenfalls in Gegenwart eines Verdünnungsmittels und gegebenenfalls in Gegenwart eines Säurebindemittels umsetzt, und daß man gegebenenfalls deren Salze erhält, wenn man Pyranylalkyl-benzyl-ether der Formel (I) mit Salzen nach üblichen Methoden umsetzt.

Schließlich wurde gefunden, daß die neuen Pyranylalkylbenzyl-ether der allgemeinen Formel (I) herbizide, insbesondere auch selektiv-herbizide Eigenschaften besitzen.

Überraschenderweise zeigen die erfindungsgemäßen Pyranylalkyl-benzyl-ether der allgemeinen Formel (I) bei vergleichbar guter herbizider Wirkung gegen Unkräuter eine erheblich höhere Selektivität gegenüber wichtigen Kulturpflanzen, als die aus dem Stand der Technik bekannten Benzylether, wie beispielsweise der (2-Methoxycyclohexyl)-(2-fluor-benzyl)-ether, welche chemisch und wirkungsmäßig naheliegende Verbindungen sind.

Die erfindungsgemäßen Pyranylalkyl-benzylether sind durch die Formel (I) allgemein definiert. Bevorzugt sind Verbindungen der Formel (I), bei welchen

$R^1$ und $R^2$ unabhängig voneinander jeweils für Wasserstoff oder für geradkettiges oder verzweigtes Alkyl mit 1 bis 4 Kohlenstoffatomen stehen,

Py für einen Rest

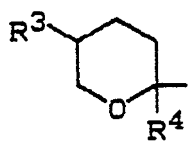

oder für einen Rest

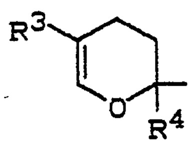

steht, wobei

$R^3$ für Wasserstoff oder für geradkettiges oder verzweigtes Alkyl mit 1 bis 8 Kohlenstoffatomen steht und

$R^4$ für geradkettiges oder verzweigtes Alkyl mit 1 bis 8 Kohlenstoffatomen, für geradkettiges oder verzweigtes Aralkyl mit 6 bis 10 Kohlenstoffatomen im Arylteil und 1 bis 4 Kohlenstoffatomen im Alkylteil, für geradkettiges oder verzweigtes Alkoxyalkyl mit jeweils 1 bis 4 Kohlenstoffatomen in den einzelnen Alkylteilen oder für geradkettiges oder verzweigtes Benzyloxyalkyl mit 1 bis 4 Kohlenstoffatomen im Alkylteil steht und

Ar für jeweils gegebenenfalls einfach oder mehrfach, gleich oder verschieden substituiertes Phenyl, Naphthyl, 2-Pyridyl, 4-Pyridyl, 2-Pyrimidyl oder 4-Pyrimidyl steht, wobei als Substituenten jeweils in Frage kommen: Halogen, Cyano, Nitro, Hydroxycarbonyl sowie jeweils geradkettiges oder verzweigtes Alkyl, Alkoxy, Alkoxycarbonyl oder Halogenalkyl mit jeweils 1 bis 4 Kohlenstoffatomen im Alkylteil und im Fall des Halogenalkyl 1 bis 9 gleichen oder verschiedenen Halogenatomen,

sowie für den Fall, daß Ar für Pyridyl oder Pyrimidyl steht, auch deren Hydrochlorid-Salze.

Besonders bevorzugt sind Verbindungen der Formel (I), bei welchen

$R^1$ und $R^2$ unabhängig voneinander jeweils für Wasserstoff, Methyl, Ethyl, n-oder i-Propyl und n-, i-, s-, oder t-Butyl stehen,

Py für einen Rest

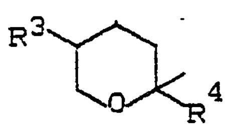

oder für einen Rest

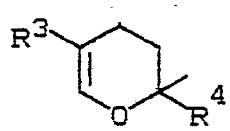

steht, wobei

$R^3$ für Wasserstoff, Methyl, Ethyl, n-oder i-Propyl, n-, i-, s-oder t-Butyl, n-oder i-Pentyl, n-oder i-Hexyl steht,

$R^4$ für Methyl, Ethyl, n-oder i-Propyl, n-, i-, s-oder t-Butyl, n-oder i-Pentyl n-oder i-Hexyl, Benzyl, Methoxymethyl, Ethoxymethyl oder Benzyloxymethyl steht und

Ar für jeweils gegebenenfalls ein-bis dreifach, gleich oder verschieden substituiertes Phenyl, Naphthyl, 2-Pyridyl, 4-Pyridyl, 2-Pyrimidyl oder 4-Pyrimidyl steht, wobei als Substituenten jeweils in Frage kommen: Fluor, Chlor, Brom, Cyano, Nitro, Hydroxycarbonyl, Methyl, Ethyl, n-oder i-Propyl, Methoxy, Ethoxy, Methoxycarbonyl, Ethoxycarbonyl oder Trifluormethyl,

sowie für den Fall, daß Ar für Pyridyl steht, auch deren Hydrochlorid-Salze.

Im einzelnen seien außer den bei den Herstellungsbeispielen genannten Verbindungen die folgenden Pyranylalkyl-benzyl-ether der allgemeinen Formel (I) erwähnt:

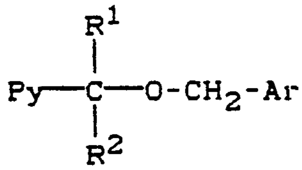

(I)

<u>Tabelle 1</u>

| Py | $R^1$ | $R^2$ | Ar |
|---|---|---|---|
| 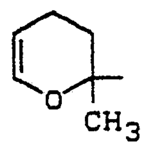 | H | H | 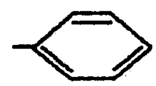 |
| 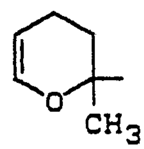 | H | H | 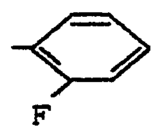 |
| 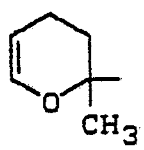 | H | H | 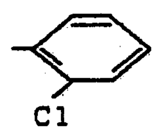 |
| 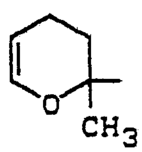 | H | H | 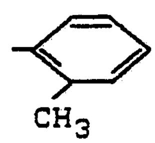 |
| 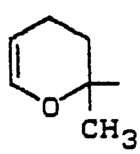 | H | H | 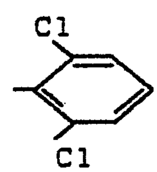 |
| 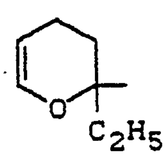 | H | H | 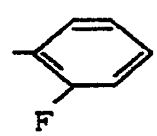 |

Tabelle 1 (Fortsetzung)

| Py | $R^1$ | $R^2$ | Ar |
|---|---|---|---|
| 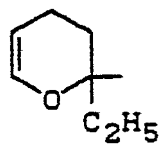 | H | H | 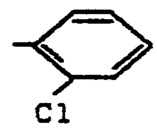 |
| 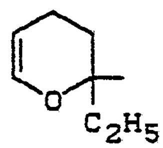 | H | H | 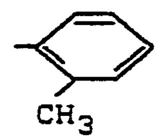 |
| 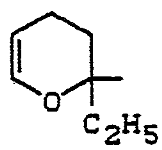 | H | H | 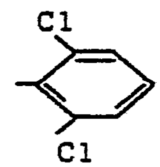 |
| 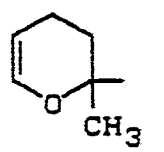 | $CH_3$ | H | 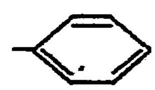 |
| 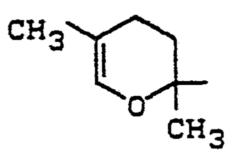 | H | H | 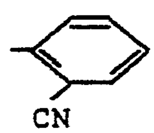 |
| 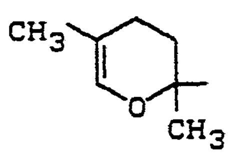 | H | H | 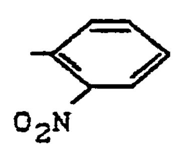 |
| 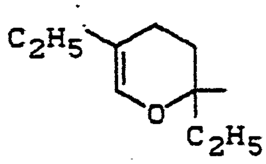 | $CH_3$ | H | 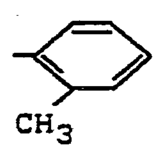 |

<u>Tabelle 1</u> (Fortsetzung)

| Py | $R^1$ | $R^2$ | Ar |
|---|---|---|---|
| 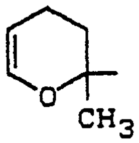 | H | H | 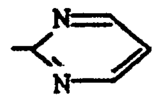 |
| 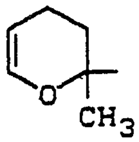 | $CH_3$ | $CH_3$ | 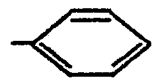 |
| 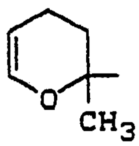 | $CH_3$ | $CH_3$ | 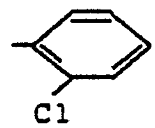 |
| 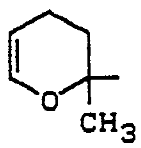 | $CH_3$ | $CH_3$ | 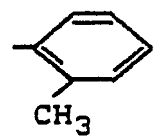 |
| 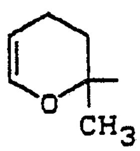 | $CH_3$ | $CH_3$ | 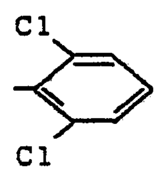 |
| 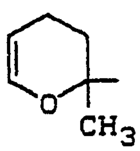 | $CH_3$ | $CH_3$ | 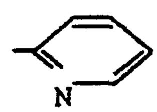 |
| 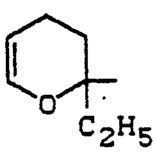 | H | H | 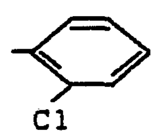 |

Tabelle 1 (Fortsetzung)

| Py | $R^1$ | $R^2$ | Ar |
|---|---|---|---|
| 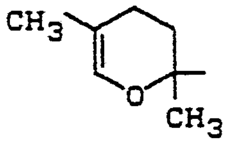 | H | H | 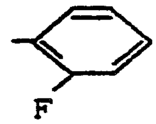 |
| 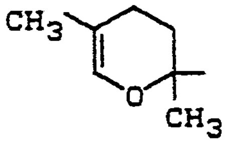 | $CH_3$ | H | 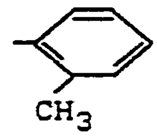 |
| 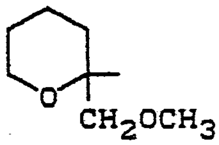 | H | H | 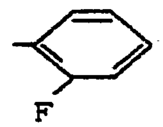 |
| 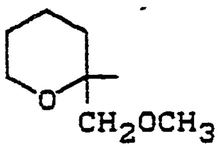 | $CH_3$ | H | 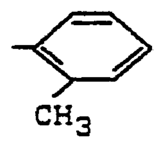 |
| 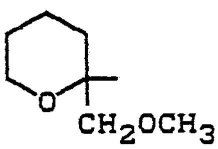 | H | H | 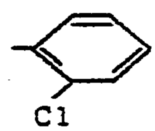 |
| 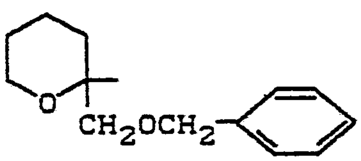 | H | H | 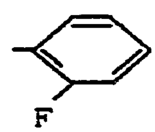 |

Verwendet man beispielsweise 2-Hydroxymethyl-2-methyltetrahydropyran und 2,6-Dichlorbenzylbromid als Ausgangsstoffe, so läßt sich der Reaktionsablauf durch das folgende Formelschema darstellen:

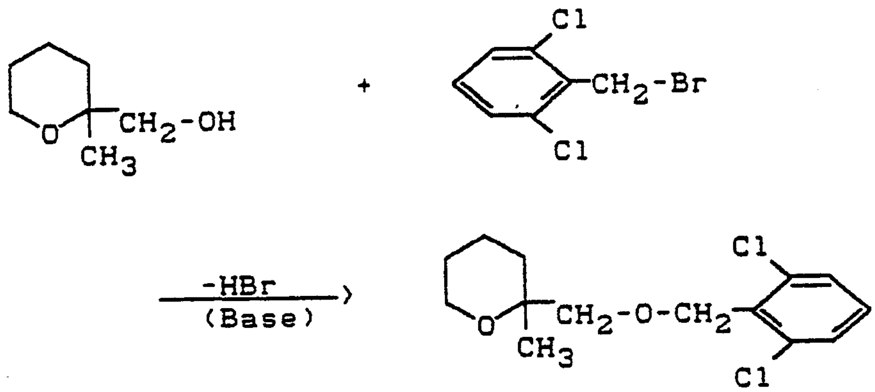


Die zur Durchführung des erfindungsgemäßen Verfahrens als Ausgangsstoffe benötigten Pyranylalkylcarbinole sind durch die Formel (II) allgemein definiert. In dieser Formel (II) stehen $R^1$, $R^2$ und Py vorzugsweise für diejenigen Reste, die bereits im Zusammenhang mit der Beschreibung der erfindungsgemäßen Stoffe der Formel (I) als bevorzugt für diese Substituenten genannt wurden.

Die Pyranylalkylcarbinole der Formel (II) sind teilweise bekannt (vgl. z.B. US-PS 4 388 104; J.Chem.Soc., Perkin Trans. I. 1981, 3034-3040; US-PS 3 122 587).

Die Pyranylalkylcarbinole der Formel (IIa),

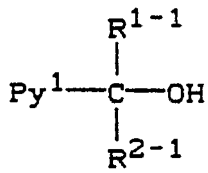

(IIa)

in welcher

$R^{1-1}$ und $R^{2-1}$ unabhängig voneinander jeweils für Wasserstoff oder Alkyl stehen,

$Py^1$ für einen Rest

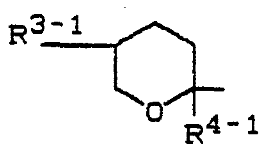

oder für einen Rest

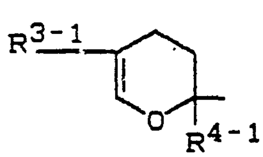

steht, wobei

$R^{3-1}$ für Wasserstoff oder Alkyl steht,

$R^{4-1}$ für Alkyl, Aralkyl, Alkoxyalkyl oder Aralkoxyalkyl steht,

ausgenommen sind jedoch die Verbindungen der Formeln

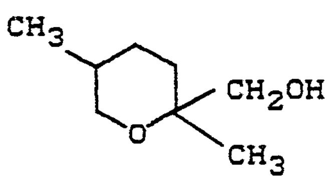 , 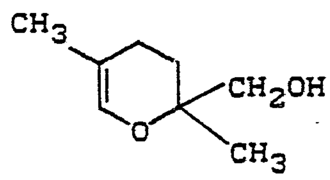 und 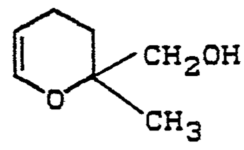

sind neu.

Bevorzugt sind Verbindungen der Formel (IIa), in welcher

$R^{1-1}$ und $R^{2-1}$ unabhängig voneinander jeweils für Wasserstoff oder für geradkettiges oder verzweigtes Alkyl mit 1 bis 4 Kohlenstoffatomen stehen,

$Py^1$ für einen Rest

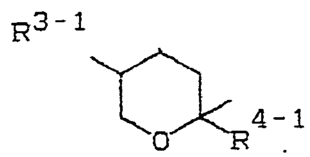

oder für einen Rest

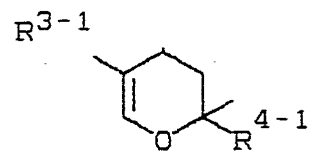

steht, wobei

$R^{3-1}$ für Wasserstoff oder für geradkettiges oder verzweigtes Alkyl mit 1 bis 8 Kohlenstoffatomen steht und

$R^{4-1}$ für geradkettiges oder verzweigtes Alkyl mit 1 bis 8 Kohlenstoffatomen, für geradkettiges oder verzweigtes Aralkyl mit 6 bis 10 Kohlenstoffatomen im Arylteil und 1 bis 4 Kohlenstoffatomen im Alkylteil, für geradkettiges oder verzweigtes Alkoxyalkyl mit jeweils 1 bis 4 Kohlenstoffatomen in den einzelnen Alkylteilen oder für geradkettiges oder verzweigtes Benzyloxyalkyl mit 1 bis 4 Kohlenstoffatomen im Alkylteil steht,

ausgenommen sind jedoch die Verbindungen der Formeln

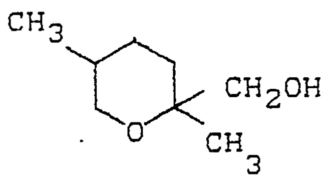 , 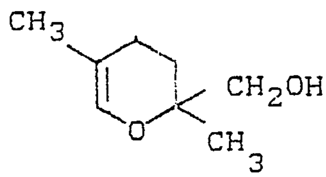 und 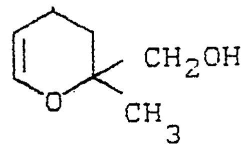

Besonders bevorzugt sind Verbindungen der Formel (IIa), in welcher

$R^{1-1}$ und $R^{2-1}$ unabhängig voneinander jeweils für Wasserstoff, Methyl, Ethyl, n-oder i-Propyl sowie n-, i-, s-, oder t-Butyl stehen,

$Py^1$ für einen Rest

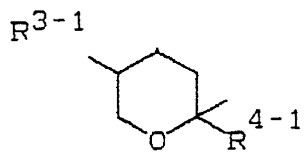

oder für einen Rest

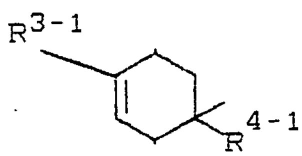

steht, wobei

$R^{3-1}$ für Wasserstoff, Methyl, Ethyl, n-oder i-Propyl, n-, i-, s-oder t-Butyl, n-oder i-Pentyl sowie n-oder i-Hexyl steht,

$R^{4-1}$ für Methyl, Ethyl, n-oder i-Propyl, n-, i-, s-oder t-Butyl, n-oder i-Pentyl, n-oder i-Hexyl, Benzyl, Methoxymethyl, Ethoxymethyl oder Benzyloxymethyl steht,

ausgenommen sind jedoch die Verbindungen der Formeln

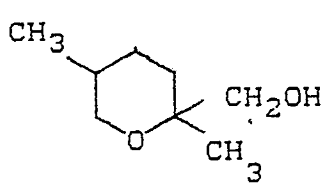 , 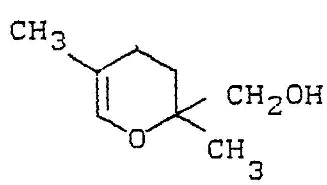 und 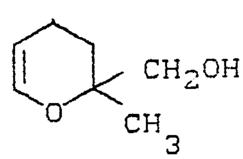 .

Man erhält die neuen Pyranylalkylcarbinole, wenn man Acrolein-Derivate der Formel (IV),

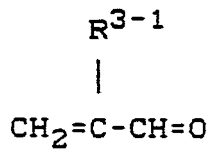

(IV)

in welcher

$R^{3-1}$ die oben angegebene Bedeutung hat, mit Acrylsäureester-Derivaten der Formel (Va),

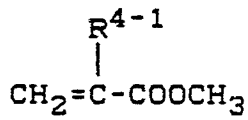

(Va)

in welcher

$R^{4-1}$ die oben angegebene Bedeutung hat,

oder mit Acrolein-Derivaten der Formel (Vb),

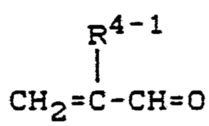

(Vb)

in welcher

$R^{4-1}$ die oben angegebene Bedeutung hat, in einer ersten Stufe in prinzipiell bekannter Art und Weise cyclisiert (vgl. z.B. Bull. Soc. Chim. France 1977, 983) zu den Dihydropyranylverbindungen der Formel (VIa),

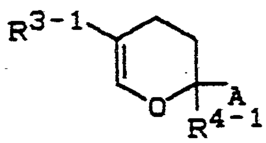

(VIa)

in welcher

A für eine Methoxycarbonylgruppe oder eine Aldehydgruppe steht und

$R^{3-1}$ und $R^{4-1}$ die oben angegebene Bedeutung haben,

und bei diesen Verbindungen die Doppelbindung im Pyranyl-Ring gegebenenfalls in einer zweiten Stufe in bekannter Weise katalytisch hydriert (vgl. z.B. Zh. Org. Khim. 12, 325 [1976]);

und die so erhältlichen Dihydro-oder Tetrahydropyranylderivate der Formel (VI),

$Py^1$-A (VI)

in welcher

A und $Py^1$ die oben angegebenen Bedeutungen haben, nach üblichen Verfahren mit komplexen Hydriden, wie beispielsweise Lithiumaluminiumhydrid, reduziert und auf diese Weise Carbinole der Formel (IIb) erhält

$Py^1$-$CH_2$-OH(IIb)

in welcher

$Py^1$ die oben angegebene Bedeutung hat.

Es ist auch möglich die Hydrierung der Doppelbindung und die Reduktion der Ester bzw. Aldehydgruppierung (Substituent A) in einem Reaktionsschritt durchzuführen, indem man beispielsweise unter Verwendung von Raney-Nickel

als Katalysator bei Temperaturen zwischen 120° C und 180° C und einem Druck zwischen 180 und 250 bar hydriert [vgl. Monatshefte für Chemie 102, 1081 (1971) und die Herstellungsbeispiele].

Führt man die Reduktion der Ester bzw. Aldehydgruppierung der Pyranyl-Derivate der Formel -(VI) mit metallorganischen Verbindungen, wie beispielsweise Methylmagnesiumiodid oder Butyllithium, -nach üblichen Verfahren -durch, so erhält man gleichzeitig alkylierte Carbinole der Formel (IIc),

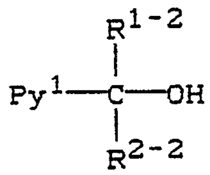

(IIc)

in welcher

$Py^1$ die oben angegebene Bedeutung hat und

$R^{1-2}$ und $R^{2-2}$ für Alkyl stehen.

Man erhält Pyranylcarbinole der Formel (IId),

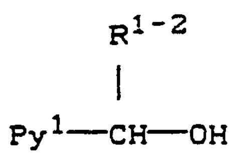

(IId)

in welcher

$Py^1$ und $R^{1-2}$ die oben angegebene Bedeutung haben,

wenn man ebenfalls in Analogie zu bekannten Verfahren, Pyranylcarbonsäureester der Formel (VIb)

$Py^1$-$COOCH_3$ (VIb)

in welcher

$Py^1$ die oben angegebene Bedeutung hat,

zunächst in einer 1.Stufe in allgemein bekannter Weise mit Säuren oder Basen an der Estergruppe verseift (vgl. z.B. J.Amer.Chem.Soc. 104, 2490 -[1982]) und die so erhältlichen Pyranylcarbonsäuren der Formel (VII),

$Py^1$-COOH (VII)

in welcher

$Py^1$ die oben angegebene Bedetung hat,

in einer 2. Stufe

mit Alkyllithiumverbindungen der Formel (VIII)

$R^{1-2}$-Li (VIII)

in welcher

$R^{1-2}$ für Alkyl steht,

unter gleichzeitiger Alkylierung in allgemein bekannter Weise reduziert und die so erhältlichen Ketone der Formel (IX),

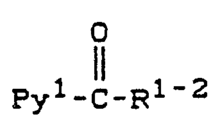

(IX)

in welcher

$Py^1$ und $R^{1-2}$ die oben angegebene Bedeutung haben in einer 3. Stufe ebenfalls in allgemein bekannter Weise beispielsweise mit Natriumborhydrid reduziert zu den entsprechenden Carbinol-Derivaten der Formel (IId) (vgl. auch die Herstellungsbeispiele). Auf analoge Weise lassen sich auch die bekannten Pyranylalkylcarbinole der Formel II herstellen.

Die Acrolein-Derivate der Formeln (IV) bzw. -(Vb), die Acrylsäureester-Derivate der Formel (Va) und die Alkyllithiumverbindungen der Formel (VIII) sind allgemein bekannte Verbindungen der organischen Chemie oder lassen sich in Analogie zu bekannten Verbindungen herstellen.

Die zur Durchführung des erfindungsgemäßen Verfahrens weiterhin als Ausgangsstoffe benötigten Arylalkylhalogenide sind durch die Formel (III) allgemein definiert. In dieser Formel (III) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Hal steht vorzugsweise für Chlor, Brom oder Iod. Die Arylalkylhalogenide der Formel (III) sind allgemein bekannte Verbindungen der organischen Chemie.

Als Salze verwendet man bei dem erfindungsgemäßen Verfahren beispielsweise Alkali-oder Erdalkalihydroxide, -chloride, -carbonate, -hydrogencarbonate, -sulfate oder -phosphate oder Mineralsäuren wie Chlorwasserstoffsäure.

Als Verdünnungsmittel zur Durchführung des erfindungsgemäßen Verfahrens kommen inerte organische Lösungsmittel in Frage. Vorzugsweise verwendet man aliphatische, cyclische oder aromatische Kohlenwasserstoffe, wie Benzin, Benzol, Toluol, Xylol, Petrolether, Hexan, Cyclohexan, Ether wie Diethylether, Dioxan oder Tetrahydrofuran, Amide wie Dimethylformamid, Dimethylacetamid, N-Methylformanilid, N-Methylpyrrolidon oder Hexamethylphosphorsäuretriamid oder Nitrile wie Acetonitril oder Propionitril.

Das erfindungsgemäße Verfahren wird vorzugsweise in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxdid oder Kalium-hydroxid, Alkalimetallcarbonate, wie Natriumcarbonat, Kaliumcarbonat oder Natriumhydrogencarbonat, Alkalimetallhydride, wie Natriumhydrid, oder Alkalimetallalkoholate, wie Natriummethylat, Natriumethylat oder Kalium-tert.-butylat.

Die Reaktionstemperaturen können bei der Durchführung des erfindungsgemäßen Verfahrens in einem größeren Bereich variiert werden. Im allgemeinen arbeitet man bei Temperaturen zwischen -20° C und +100° C, vorzugsweise bei Temperaturen zwischen 0° C und 80° C.

Zur Durchführung des erfindungsgemäßen Verfahrens setzt man pro Mol an Pyranylalkylcarbinol der Formel (II) im allgemeinen 1,0 bis 1,5 Mol, vorzugsweise 1,0 bis 1,2 Mol an Arylalkylhalogenid der Formel (III) und 1,0 bis 1,5 Mol, vorzugsweise 1,0 bis 1,2 Mol an Säurebindemittel ein. Die Reaktionsdurchführung, Aufarbeitung und Isolierung der Reaktionsprodukte erfolgt nach allgemein bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso

können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von monokotylen Unkräutern in dikotylen und monokotylen Kulturpflanzen wie beispielsweise Getreide, Reis oder Mais einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Besonders geeignete Mischpartner sind auch Herbizide aus den Klassen der Nitroaniline, der Chloracetanilide, der Heteroaryloxyacetamide, der Cyclohexandione, der Harnstoffe, der Pyrimidone sowie der Aryloxy-oder Heteroaryloxyphenoxypropionsäurederivate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

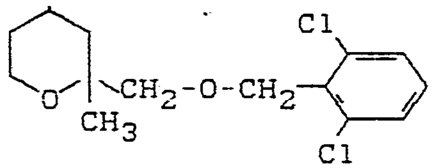

Zu 6,2 g (0,055 Mol) Kalium-tert.-butylat in 100 ml trokkenem Tetrahydrofuran gibt man unter Rühren 6,5 g (0,05 Mol) 2-Hydroxymethyl-2-methyl-tetrahydropyran, rührt nach beendeter Zugabe weitere 15 Minuten bei Raumtemperatur, setzt dann 12,0 g (0,05 Mol) 2,6-Dichlor-benzylbromid zu, rührt weitere 3 Stunden bei Raumtemperatur und saugt den ausgefallenen Feststoff ab. Das Filtrat wird im Vakuum eingeengt, der Rückstand in Dichlormethan aufgenommen, zweimal mit je 100 ml Wasser gewaschen, über Natriumsulfat getrocknet und im Vakuum vom Lösungsmittel befreit.

Man erhält 13,2 g (91 % der Theorie) an (2-Methyl-tetrahydropyran-2-ylmethyl)-(2,6-dichlorbenzyl)-ether als Öl.

$^1$H-NMR ($CDCl_3$) ε(ppm) : 1,2 (S, 3H); 4,8 - (S,2H)

Herstellung der Ausgangsverbindung

Beispiel II-I

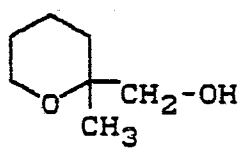

Zu 158 g (1,0 Mol) 2-Methyltetrahydropyran-2-yl-carbonsäuremethylester in 1000 ml absolutem Ether tropft man unter Rühren und einer Stickstoff-Atmosphäre bei 0°C bis 5°C langsam eine Suspension von 26,7 g (0,7 Mol) Lithiumaluminiumhydrid in Ether. Nach beendeter Zugabe rührt man weitere 60 Minuten bei 0°C und gibt dann tropfenweise zunächst 50 ml Essigsäuremethylester und dann 1500 ml 2-normale wässrige Schwefelsäure zu. Zur Aufarbeitung trennt man die organische Phase ab, sättigt die wässrige Phase mit Natriumchlorid und extrahiert dreimal mit jeweils 200 ml Ether. Die vereinigten organischen Phasen werden mit gesättigter wässriger Natriumchloridlösung gewaschen, über Natriumsulfat getrocknet und destilliert.

Man erhält 107 g (82 % der Theorie) an 2-Hydroxymethyl-2-methyl-tetrahydropyran vom Siedepunkt 76°C bei 13,3 mbar und vom Brechungsindex $n_D^{20}$ 1,4600.

Beispiel 2

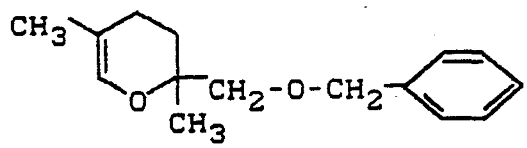

Zu 6,0 g (0,2 Mol) Natriumhydrid (80-prozentig in Paraffinöl) in 200 ml trocknenem Dimethylformamid tropft man unter Rühren bei Raumtemperatur 28,4 g (0,2 Mol) 2-Hydroxymethyl-2,5-dimethyl-2,3-dihydro-4H-pyran, rührt nach beendeter Zugabe weitere 60 Minuten bei Raumtemperatur, tropft dann 25,2 g (0,2 Mol) Benzylchlorid zu und rührt weitere 4 Stunden bei Raumtemperatur. Zur Aufarbeitung gießt man die Reaktionsmischung in Wasser, extrahiert mehrmals mit Dichlormethan, wäscht die vereinigten organischen Phasen mit Wasser, trocknet über Natriumsulfat und destilliert.

Man erhält 28 g (61 % der Theorie) an (2,5-dimethyl-2,3-dihydro-4H-pyran-2-ylmethyl)-benzylether vom Siedepunkt 130°C bei 1,6 mbar.

In entsprechender Weise und gemäß den allgemeinen Angaben zur Herstellung erhält man die folgenden Pyranyl-alkyl-benzyl-ether der allgemeinen Formel (I):

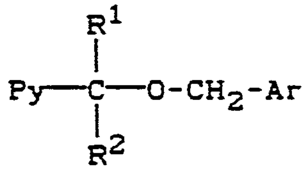 (I)

Tabelle 2

| Bsp. Nr. | Py | $R^1$ | $R^2$ | Ar | physikalische Eigenschaften |
|---|---|---|---|---|---|
| 3 | 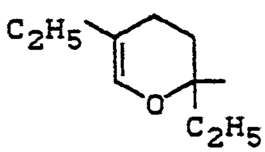 | H | H | 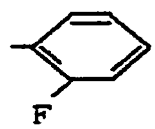 | Kp 100°C/ 0,3 mbar |
| 4 | 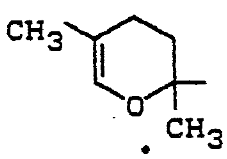 | H | H | 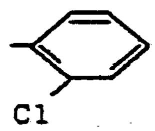 | Kp 125°C/ 0,7 mbar |
| 5 | 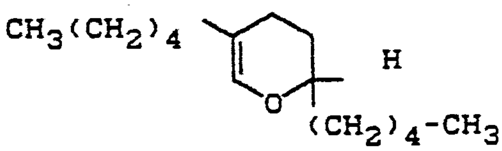 | H | H | 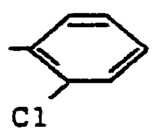 | Kp 153°C/ 0,25 mbar |
| 6 | 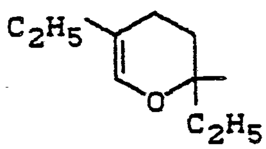 | H | H | 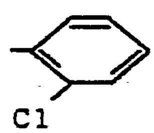 | Kp 126°C/ 0,25 mbar |

Tabelle 2 (Fortsetzung)

| Bsp. Nr. | Py | $R^1$ | $R^2$ | Ar | physikalische Eigenschaften |
|---|---|---|---|---|---|
| 7 | 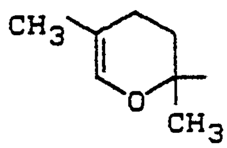 | H | H | 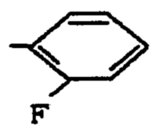 | Kp 94°C/ 0,5 mbar |
| 8 | 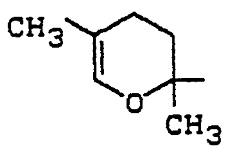 | H | H | 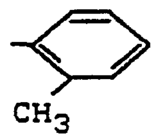 | NMR*): 1,6 (d,3H I=1Hz) |
| 9 | 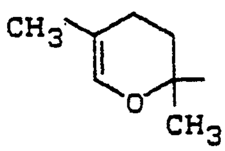 | H | H | 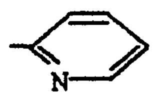 | NMR*): 1,55 (d,3H I=1Hz) |
| 10 | 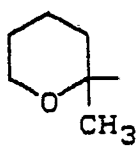 | H | H | 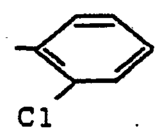 | NMR*): 1,25 (s,3H) |
| 11 | 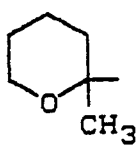 | H | H | 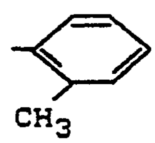 | NMR*):1,2 (s,3H) |
| 12 | 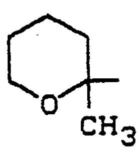 | H | H | 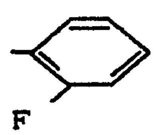 | NMR*): 1.25 (s,3H) |

Tabelle 2 (Fortsetzung)

| Bsp. Nr. | Py | $R^1$ | $R^2$ | Ar | physikalische Eigenschaften |
|---|---|---|---|---|---|
| 13 | 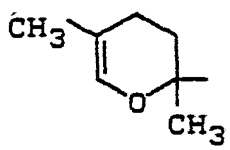 | H | H | 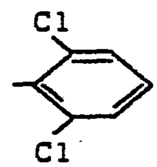 | NMR*): 1,55 (d,3H, I=1Hz) |
| 14 | 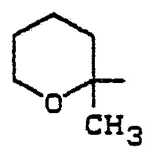 | $CH_3$ | H | 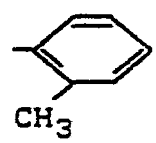 | NMR*): 2,35 (s,3H) |
| 15 | 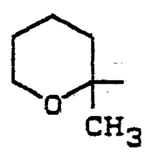 | $CH_3$ | $CH_3$ | 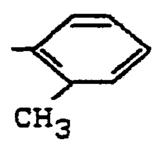 | Kp 135-137°C/ 3,3 mbar |
| 16 | 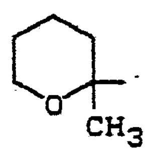 | $CH_3$ | H | 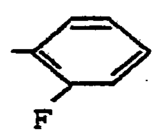 | NMR*): 4,65 (s,2H) |
| 17 | 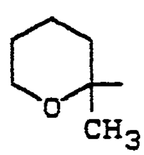 | $CH_3$ | $CH_3$ | 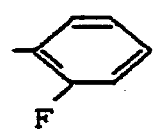 | Kp 133°-134°C/ 3,3 mbar |
| 18 | 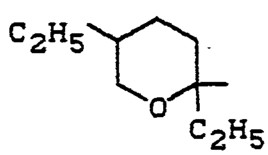 | H | H | 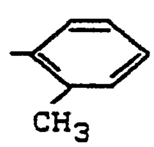 | NMR*): 2.35 (s,3H) |

<u>Tabelle 2</u> (Fortsetzung)

| Bsp. Nr. | Py | $R^1$ | $R^2$ | Ar | physikalische Eigenschaften |
|---|---|---|---|---|---|
| 19 | 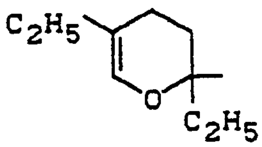 | H | H | 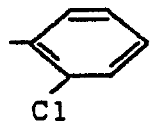 | Kp 127°C/ 0,3 mbar |
| 20 | 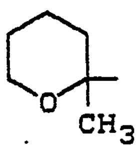 | H | H | 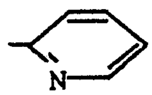 | NMR*): 1,25 (s,3H) |
| 21 | 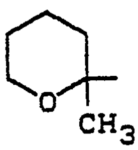 | H | H | 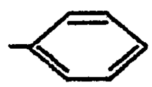 | NMR*): 1,25 (s,3H) |
| 22 | 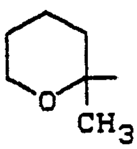 | $CH_3$ | H | 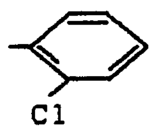 | NMR*): 4,65 (s,2H) |
| 23 | 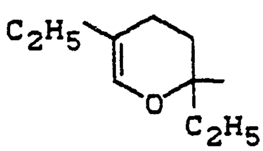 | H | H | 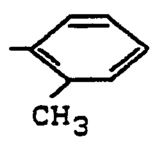 | Kp 130°C/ 0,9 mbar |
| 24 | 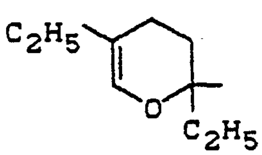 | H | H | 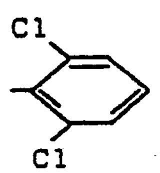 | Kp 149°C/ 0,9 mbar |

<u>Tabelle 2</u> (Fortsetzung)

| Bsp. Nr. | Py | $R^1$ | $R^2$ | Ar | physikalische Eigenschaften |
|---|---|---|---|---|---|
| 25 | 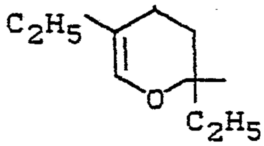 | H | H | 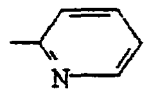 | Kp 133° C/ 1,0 mbar |
| 26 | 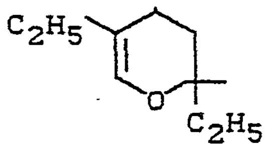 | H | H | 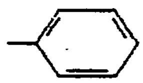 | Kp 133° C/ 1,0 mbar |
| 27 | 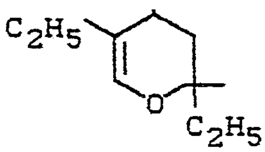 | H | H | 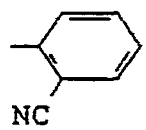 | NMR*): 3,45 (s,2H) |
| 28 | 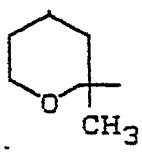 | H | H | 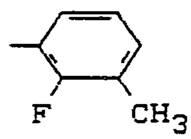 | Kp 144° C/ 1,0 mbar |
| 29 | 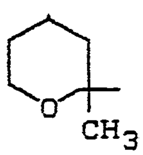 | H | H | 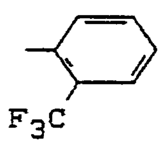 | Kp 135° C/ 0,9 mbar |
| 30 | 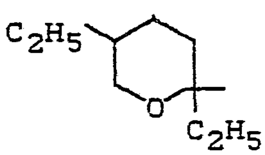 | H | H | 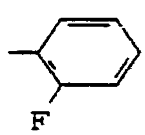 | Kp 150° C/ 0,9 mbar |

<u>Tabelle 2</u> (Fortsetzung)

| Bsp. Nr. | Py | $R^1$ | $R^2$ | Ar | physikalische Eigenschaften |
|---|---|---|---|---|---|
| 31 | 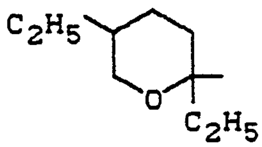 | H | H | 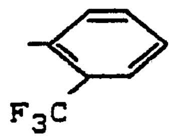 | Kp 145°C/ 1,5 mbar |
| 32 | 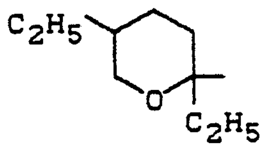 | H | H | 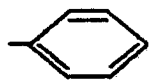 | Kp 154°C/ 0,5 mbar |
| 33 | 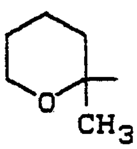 | $CH_3$ | H | 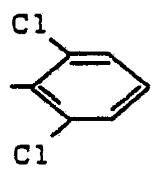 | Kp 110°C/ 0,9 mbar |
| 34 | 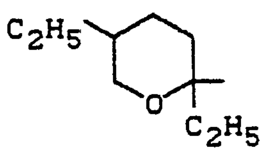 | H | H | 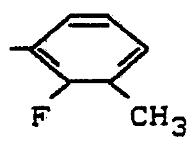 | Kp 164°C/ 0,9 mbar |
| 35 | 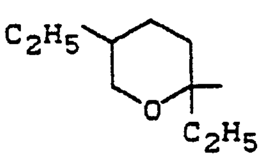 | H | H | 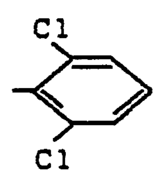 | Kp 175°C/ 2,5 mbar |
| 36 | 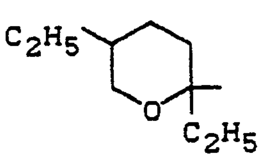 | H | H | 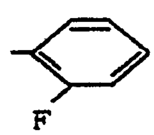 | Kp 150°C/ 0,9 mbar |

<u>Tabelle 2</u> (Fortsetzung)

| Bsp. Nr. | Py | $R^1$ | $R^2$ | Ar | physikalische Eigenschaften |
|---|---|---|---|---|---|
| 37 | 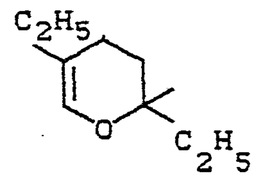 | H | H | 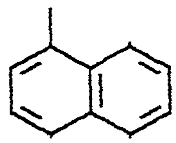 | NMR*):3,4 (s, 2H) |
| 38 | 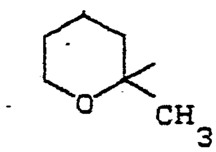 | $CH_3$ | H | 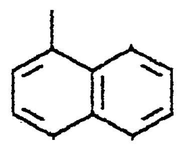 | NMR*):5,0 (s, 2H) |
| 39 | 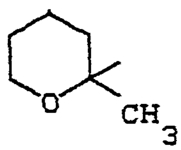 | H | H | 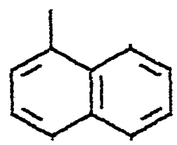 | NMR*):1,2 (s, 3H) |
| 40 | 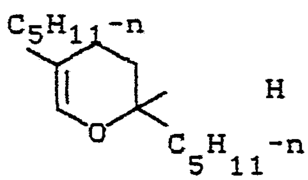 | H | H | 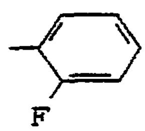 | NMR*): 3,4 (s, 2H) |
| 41 | 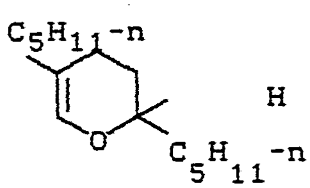 | H | H | 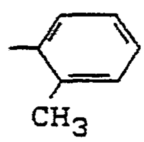 | NMR*): 2,3 (s, 2H) |
| 42 | 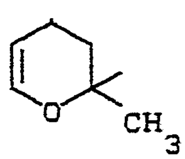 | H | H | 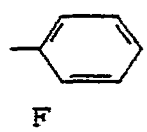 | Kp 94° C/0,3mbar |
| 43 | 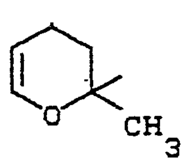 | H | H | 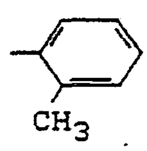 | Kp 99° C/0,2mbar |

Tabelle 2 (Fortsetzung)

| Bsp. Nr. | Py | $R^1$ | $R^2$ | Ar | physikalische Eigenschaften |
|---|---|---|---|---|---|
| 44 | 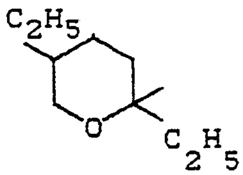 | H | H | 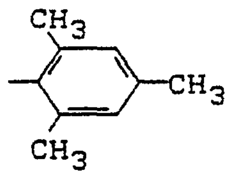 | Kp. 135° C /0,2mbar |
| 45 | 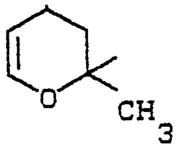 | H | H | 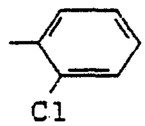 | Kp: 108° C/0,2mbar |
| 46 | 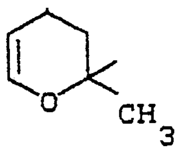 | H | H | 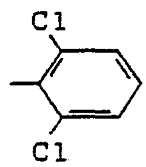 | Kp: 122° C /0,3mbar |
| 47 | 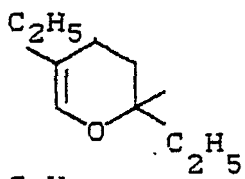 | H | H | 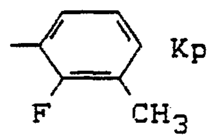 | Kp: 128° C /0,2mbar |
| 48 | 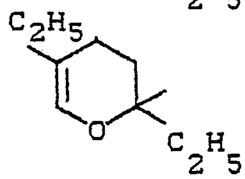 | H | H | 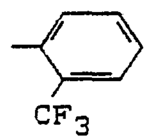 | Kp: 118° C /0,3mbar |
| 49 | 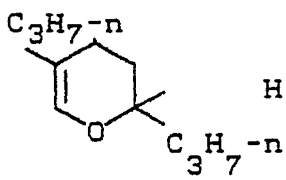 | H | H | 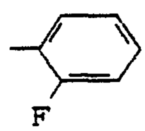 | Kp: 137° C /0,3mbar |
| 50 | 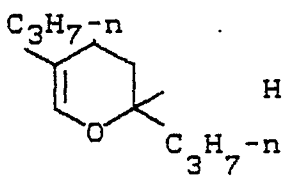 | H | H | 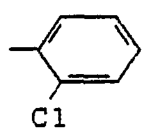 | Kp 151° C /0,2mbar |

Tabelle 2 (Fortsetzung)

| Bsp. Nr. | Py | $R^1$ | $R^2$ | Ar | physikalische Eigenschaften |
|---|---|---|---|---|---|
| 51 | 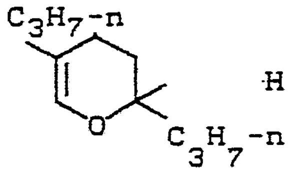 | H | H | 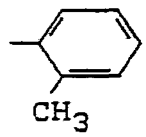 | Kp: 145° C /0,2mbar |
| 52 | 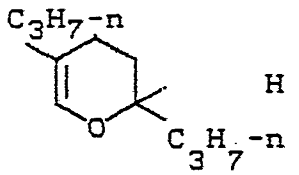 | H | H | 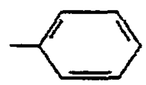 | Kp: 137° C /0,3mbar |
| 53 | 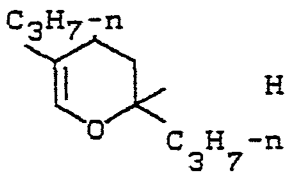 | H | H | 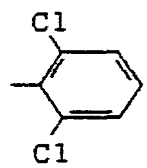 | Kp: 159° C /0,2mbar |
| 54 | 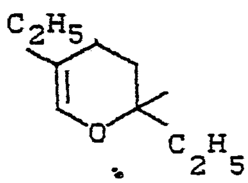 | H | H | 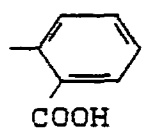 | NMR*:3,5 (s, 2H) |
| 55 | 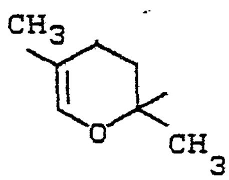 | H | H | 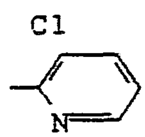 x HCl | NMR*): 4,9 (s, 2H) |
| 56 | 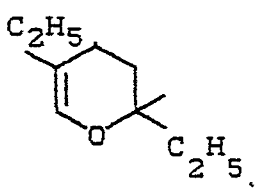 | H | H | 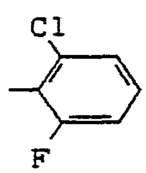 | Kp: 123° C /0,1mbar |
| 57 | 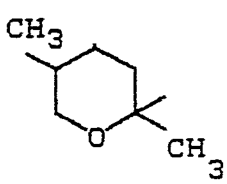 | H | H | 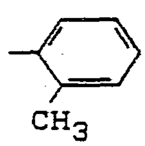 | Kp: 124-6° C /1mbar |

Tabelle 2 (Fortsetzung)

| Bsp. Nr. | Py | $R^1$ | $R^2$ | Ar | physikalische Eigenschaften |
|---|---|---|---|---|---|
| 58 | 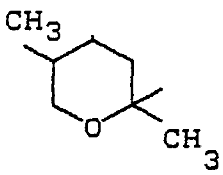 | H | H | 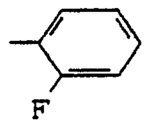 | NMR*):4,7 (s, 2H) |
| 59 | 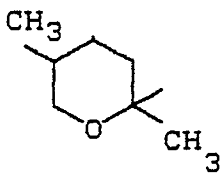 | H | H | 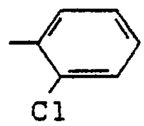 | Kp: 112° C /0,1mbar |
| 60 | 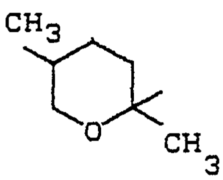 | H | H | 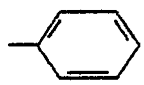 | NMR*):3,3 (s, 2H) |
| 61 | 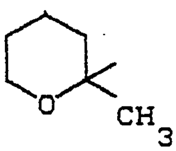 | $CH_3$ | H | 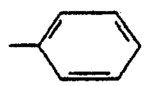 | Kp: 114° C /0,9mbar |
| 62 | 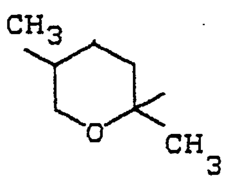 | H | H | 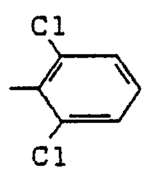 | Fp. 71-72° C |
| 63 | 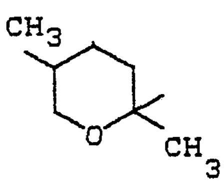 | $CH_3$ | H | 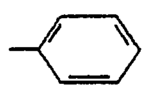 | NMR*):7,2-7,4 (m,5H) |
| 64 | 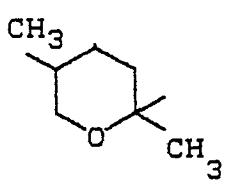 | $CH_3$ | H | 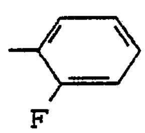 | Kp: 107° C /0,3mbar |

<u>Tabelle 2</u> (Fortsetzung)

| Bsp. Nr. | Py | $R^1$ | $R^2$ | Ar | physikalische Eigenschaften |
|---|---|---|---|---|---|
| 65 | 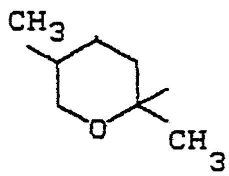 | $CH_3$ | H | 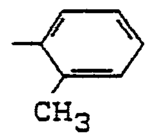 | Kp: 116-18° C /0,3mbar |
| 66 | 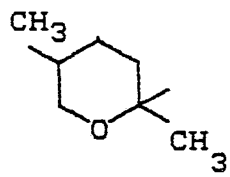 | $CH_3$ | H | 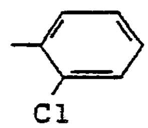 | Kp: 123° C /0,3mbar |
| 67 | 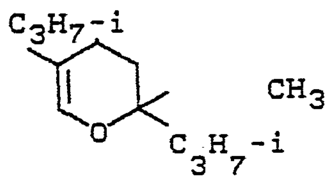 | $CH_3$ | H | 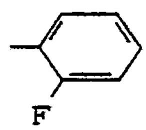 | Kp: 127° C /0,2mbar |
| 68 | 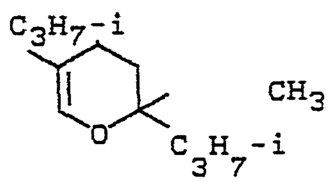 | $CH_3$ | H | 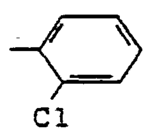 | Kp: 144° C /0,3mbar |
| 69 | 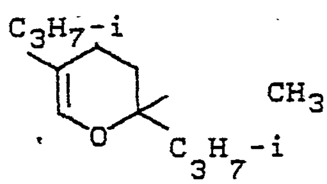 | $CH_3$ | H | 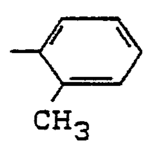 | Kp: 135° C /0,3mbar |
| 70 | 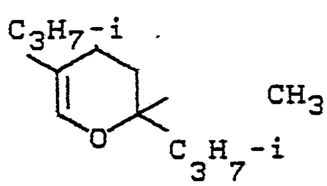 | $CH_3$ | H | 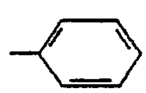 | Kp: 130° C /0,3mbar |
| 71 | 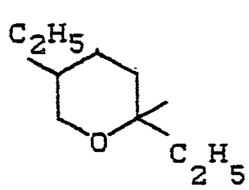 | H | H | 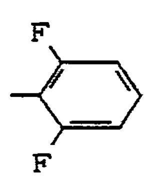 | Kp: 105-15° C /0,3-0,2mbar |

Tabelle 2 (Fortsetzung)

| Bsp. Nr. | Py | $R^1$ | $R^2$ | Ar | physikalische Eigenschaften |
|---|---|---|---|---|---|
| 72 | 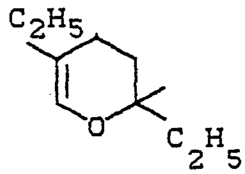 | H | H | 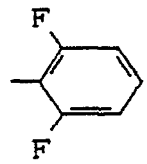 | Kp: 115° C /0,3-0,2mbar |
| 73 | 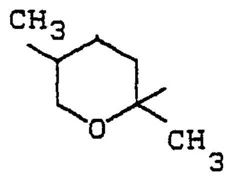 | H | H | 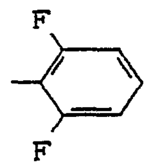 | Kp: 100° C /0,3-0,2mbar |
| 74 | 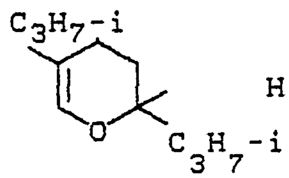 | H | H | 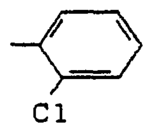 | Kp: 137° C /0,2-0,1mbar |
| 75 | 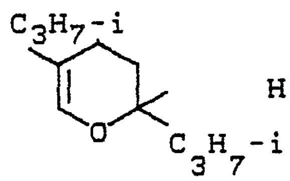 | H | H | 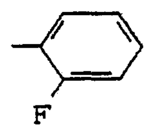 | Kp: 130-33° C /0,5mbar |
| 76 | 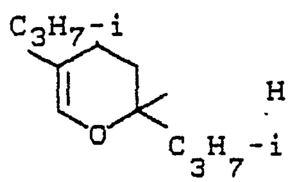 | H | H | 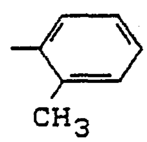 | Kp: 133-35° C /0,2mbar |
| 77 | 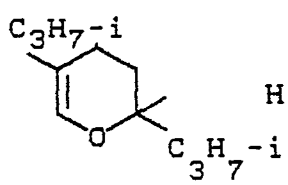 | H | H | 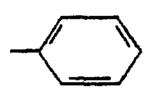 | Kp: 121-22° C /0,2mbar |
| 78 | 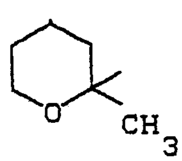 | H | H | 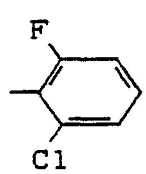 | Kp: 121-23° C /0,6mbar |

Tabelle 2 (Fortsetzung)

| Bsp. Nr. | Py | $R^1$ | $R^2$ | Ar | physikalische Eigenschaften |
|---|---|---|---|---|---|
| 79 | 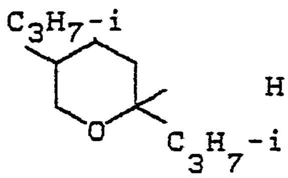 | H | H | 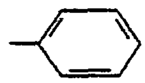 | Kp: 130-2° C /0,2mbar |
| 80 | 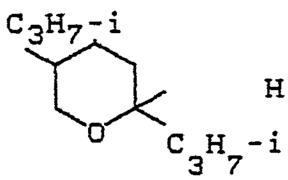 | H | H | 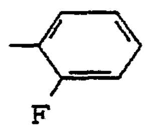 | Kp: 122-3° C /0,2mbar |
| 81 | 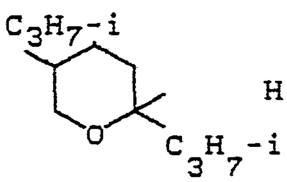 | H | H | 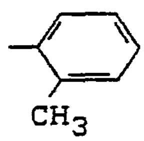 | Kp: 135-7° C /0,3mbar |
| 82 | 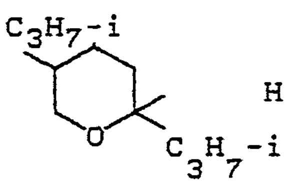 | H | H | 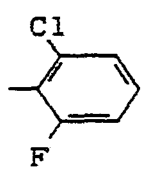 | Kp: 167-9° C /3mbar |
| 83 | 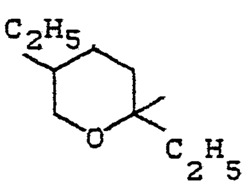 | H | H | 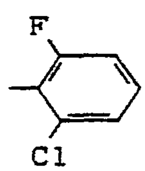 | Kp: 125-30° C /0,3mbar |
| 84 | 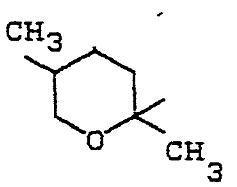 | H | H | 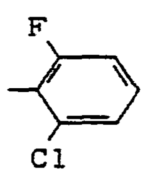 | Kp: 121° C /0,4mbar |
| 85 | 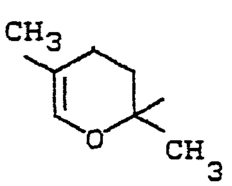 | $CH_3$ | H | 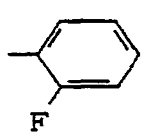 | Kp: 124-7° C /0,3mbar |

<u>Tabelle 2</u> (Fortsetzung)

| Bsp. Nr. | Py | $R^1$ | $R^2$ | Ar | physikalische Eigenschaften |
|---|---|---|---|---|---|
| 86 | 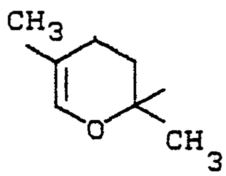 | $CH_3$ | H | 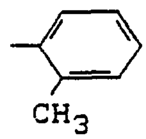 | Kp: 121-4° C /0,9mbar |
| 87 | 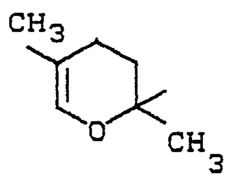 | $CH_3$ | H | 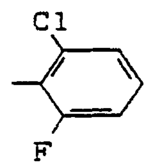 | Kp: 127-30° C /0,9mbar |
| 88 | 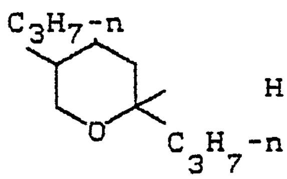 | H | H | 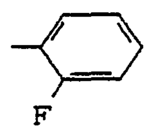 | Kp: 124° C /0,2mbar |
| 89 | 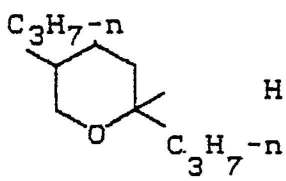 | H | H | 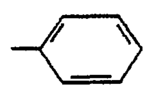 | Kp: 123° C /0,2mbar |
| 90 | 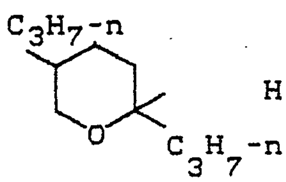 | H | H | 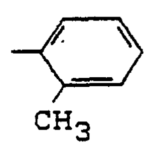 | Kp: 137° C /0,3mbar |
| 91 | 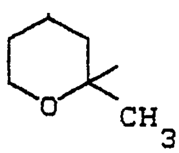 | H | H | 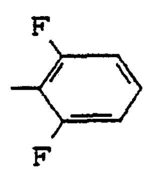 | Kp: 140-2° C /5,5mbar |
| 92 | 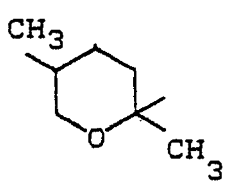 | $CH_3$ | H | 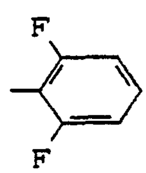 | Kp: 109-12° C /0,5mbar |

Tabelle 2 (Fortsetzung)

| Bsp. Nr. | Py | $R^1$ | $R^2$ | Ar | physikalische Eigenschaften |
|---|---|---|---|---|---|
| 93 | 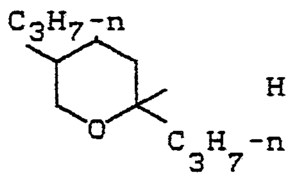 | H | H | 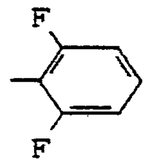 | Kp: 135° C /0,15mbar |
| 94 | 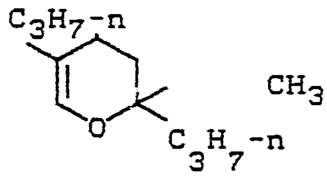 | $CH_3$ | H | 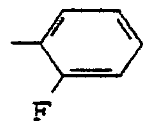 | Kp: 123° C /0,2mbar |
| 95 | 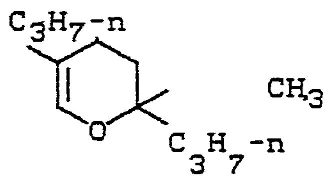 | $CH_3$ | H | 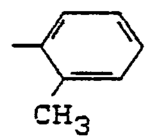 | Kp: 135° C /0,25mbar |
| 96 | 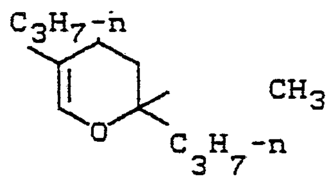 | $CH_3$ | H | 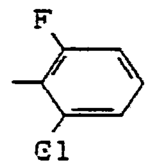 | Kp: 140° C /0,25mbar |
| 97 | 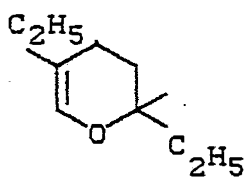 | H | H | 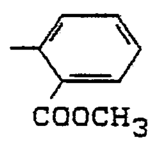 | Kp: 157° C /0,2mbar |
| 98 | 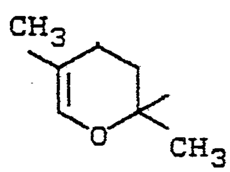 | H | H | 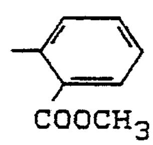 | Kp: 146° C /0,3mbar |

*) Die $^1H$-NMR-Spektren wurden in $CDCl_3$ aufgenommen. Angegeben ist die chemische Verschiebung als $\epsilon$-Wert in ppm und die Kopplungskonstante I in Hz.

Herstellung weiterer Ausgangsverbindungen

Beispiel II-2

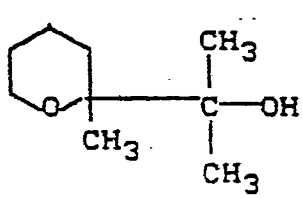

Aus 6,1 g (0,25 Mol) Magnesium und 34,8 g - (0,245 Mol) Methyliodid in Ether stellt man eine Lösung von Methylmagnesiumiodid her und tropft hierzu 15,8 g (0,1 Mol) 2-Methyl-tetrahydropyran-2-ylcarbonsäuremethylester in 100 ml Ether. Nach beendeter Zugabe erwärmt man für eine Stunde auf Rückflußtemperatur, kühlt auf 0°C ab, gibt langsam 250 ml gesättigte Ammoniumchloridlösung zu, trennt die organische Phase ab und extrahiert die wässrige Phase viermal mit jeweils 50 ml Ether. Die vereinigten Etherphasen werden mit Wasser gewaschen, über Natriumsulfat getrocknet und destilliert.

Man erhält 14,3 g (91 % der Theorie) an 2-(2-Methyltetrahydropyran-2-yl)-propan-2-ol vom Siedepunkt 80-86°C bei 13 mbar und vom Brechungsindex $n^{=0}$1,4570.

Beispiel II-3

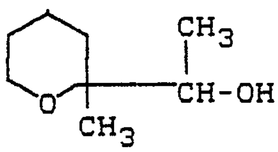

Zu einer siedenden Lösung von 61,0g (0,43 Mol) 2-Acetyl-2-methyl-tetrahydropyran in 650 ml Ethanol gibt man portionsweise 15,1 g (0,399 Mol) Natriumborhydrid und erhitzt nach beendeter Zugabe eine weitere Stunde lang auf Rückflußtemperatur. Zur Aufarbeitung engt man im Vakuum ein, versetzt mit 500 ml 1-normaler wässriger Natronlauge und extrahiert fünfmal mit jeweils 150 ml Ether. Die vereinigten Etherphasen werden nacheinander mit 150 ml 10-prozentiger wässriger Kaliumhydrogencarbonatlösung und 100 ml Wasser gewaschen, über Natriumsulfat getrocknet und destilliert.

Man erhält 51 g (83 % der Theorie) an 2-(1-Hydroxy-ethyl)-2-methyl-tetrahydropyran vom Siedepunkt 77°C-81°C bei 13 mbar und vom Brechungsindex $n^{=0}$ 1,4570.

Herstellung der Ausgangsverbindung:

Beispiel (IX-1)

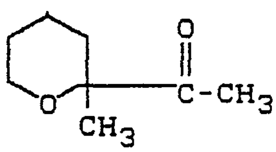

(IX-1)

Zu 40,0 g (0,277 Mol) 2-Methyltetrahydropyran-2-carbonsäure in 1000 ml absolutem Ether tropft man bei 0°C unter Rühren innerhalb von 50 Minuten 416 ml 1,6-normale Methyllithiumlösung in Hexan, läßt die Mischung auf Raumtemperatur kommen und gießt sie dann in eine Mischung von 700 g Eis und 80 ml konzentrierter Salzsäure, trennt die Etherphase ab und extrahiert die wässrige Phase noch dreimal mit jeweils 150 ml Ether. Die vereinigten Etherphasen werden nacheinander mit 100 ml 10-prozentiger Natriumcarbonatlösung und 100 ml Wasser gewaschen, über Magnesiumsulfat getrocknet und destilliert.

Man erhält 32,7 g (83 % der Theorie) an 2-Acetyl-2-methyl-tetrahydropyran vom Siedepunkt 67-70°C bei 20 mbar und vom Brechungsindex $n^{=0}$ 1,4421.

Beispiel II-4

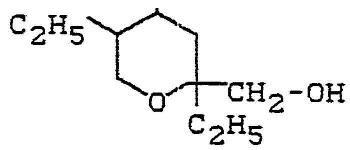

250 g (1,47 Mol) 2,5-Diethyl-2,3-dihydro-4H-pyran-2-aldehyd und 25 g Raney-Nickel in 200 ml Methanol werden 5 Stunden lang bei 150°C und 200 bar in einem Autoklaven hydriert. Zur Aufarbeitung wird das Raney-Nickel abfiltriert und das Filtrat destilliert.

Man erhält 229 g (91 % der Theorie) an 2,5-Diethyl-tetrahydropyran-2-methanol vom Siedepunkt 127°C bei 20 mbar.

Anwendungsbeispiele:

In den folgenden Anwendungsbeispielen wurde die nachstehend aufgeführte Verbindung als Vergleichsubstanz eingesetzt:

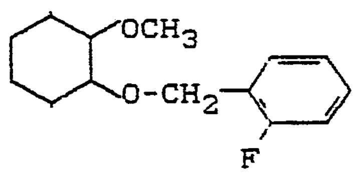

(A)

(2-Methoxycyclohexyl)-(2-fluorbenzyl)-ether (bekannt aus DE-OS 2 753 556)

Beispiel A

Pre-emergence-Test / Freiland

Lösungsmittel: 5 Gewichtsteile Aceton

Emulgator: 1 Gewichtsteil Alkylarylpolyglykolether

Zur Herstellung einer zweckmäßigen Wirkstoffzubereitung wurde 1 Gewichtsteil Wirkstoff mit der angegebenen Menge Lösungsmittel vermischt, die angegebene Menge Emulgator zugegeben und das Konzentrat mit Wasser auf die gewünschte Konzentration verdünnt.

Kurz nach dem Aussäen der Samen der Testpflanzen im Freiland wurden die einzelnen Parzellen mit einer solchen Menge der Wirkstoffzubereitung begossen, wie für eine gleichmäßige Benetzung der Bodenfläche erforderlich war. Die Wirkstoffkonzentration in der Zubereitung spielt dabei keine Rolle, entscheidend ist nur die Aufwandmenge des Wirkstoffes pro Flächeneinheit.

Nach 5 Wochen wurde der Schädigungsgrad der Testpflanzen bonitiert in % Schädigung im Vergleich zur Entwicklung der unbehandelten Kontrolle. Es bedeuten:

0 % = keine Wirkung

100 % = totale Vernichtung.

Eine deutliche Überlegenheit in der Nutzpflanzenselektivität gegenüber dem Stand der Technik zeigen in diesem Test z.B. die Verbindungen gemäß den Herstellungsbeispielen: 3 und 6.

## Ansprüche

1. Pyranylalkyl-benzyl-ether der allgemeinen Formel (I),

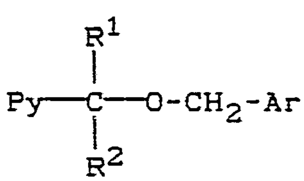

(I)

in welcher

$R^1$ und $R^2$ unabhängig voneinander jeweils für Wasserstoff oder Alkyl stehen,

Py für einen Rest

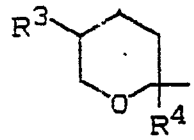

oder für einen Rest

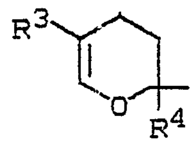

steht, wobei

$R^3$ für Wasserstoff oder Alkyl steht,

$R^4$ für Alkyl, Aralkyl, Alkoxyalkyl oder Aralkoxyalkyl steht und

Ar für jeweils gegebenenfalls substituiertes Phenyl, Naphthyl, Pyridyl oder Pyrimidyl steht,

sowie deren Salze.

2. Pyranylalkyl-benzyl-ether der Formel (I) gemäß Anspruch 1, in welcher

$R^1$ und $R^2$ unabhängig voneinander jeweils für Wasserstoff oder für geradkettiges oder verzweigtes Alkyl mit 1 bis 4 Kohlenstoffatomen stehen,

Py für einen Rest

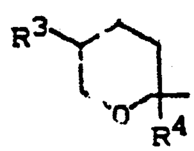

oder für einen Rest

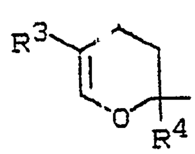

steht, wobei $R^3$ für Wasserstoff oder für geradkettiges oder verzweigtes Alkyl mit 1 bis 8 Kohlenstoffatomen steht und

$R^4$ für geradkettiges oder verzweigtes Alkyl mit 1 bis 8 Kohlenstoffatomen, für geradkettiges oder verzweigtes Aralkyl mit 6 bis 10 Kohlenstoffatomen im Arylteil und 1 bis 4 Kohlenstoffatomen im Alkylteil, für geradkettiges oder verzweigtes Alkoxyalkyl mit jeweils 1 bis 4 Kohlenstoffatomen in den einzelnen Alkylteilen oder für geradkettiges oder verzweigtes Benzyloxyalkyl mit 1 bis 4 Kohlenstoffatomen im Alkylteil steht und

Ar für jeweils gegebenenfalls einfach oder mehrfach, gleich oder verschieden substituiertes Phenyl, Naphthyl, 2-Pyri dyl, 4-Pyridyl, 2-Pyrimidyl oder 4-Pyrimidyl steht, wobei als Substituenten jeweils in Frage kommen: Halogen, Cyano, Nitro, Hydroxycarbonyl sowie jeweils geradkettiges oder verzweigtes Alkyl, Alkoxy, Alkoxycarbonyl oder Halogenalkyl mit jeweils 1 bis 4 Kohlenstoffatomen im Alkylteil und im Fall des Halogenalkyl mit 1 bis 9 gleichen oder verschiedenen Halogenatomen,

sowie für den Fall, daß Ar für Pyridyl oder Pyrimidyl steht, auch deren Hydrochlorid-Salze.

3. Pyranylalkyl-benzyl-ether der Formel (I) gemäß Anspruch 1, in welcher

$R^1$ und $R^2$ unabhängig voneinander jeweils für Wasserstoff, Methyl, Ethyl, n-oder i-Propyl und n-, i-, s-, oder t-Butyl stehen,

Py für einen Rest

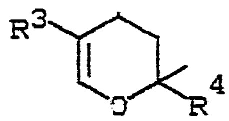

oder für einen Rest

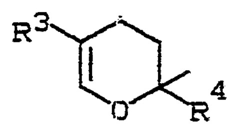

steht, wobei

$R^3$ für Wasserstoff, Methyl, Ethyl, n-oder i-Propyl, n-, i-, s-oder t-Butyl, n-oder i-Pentyl, n-oder i-Hexyl steht,

$R^4$ für Methyl, Ethyl, n-oder i-Propyl, n-, i-, s-oder t-Butyl, n-oder i-Pentyl, n-oder i-Hexyl, Benzyl, Methoxymethyl, Ethoxymethyl oder Benzyloxymethyl steht und

Ar für jeweils gegebenenfalls ein-bis dreifach, gleich oder verschieden substituiertes Phenyl, Naphthyl, 2-Pyridyl, 4-Pyridyl, 2-Pyrimidyl oder 4-Pyrimidyl steht, wobei als Substituenten jeweils in Frage kommen: Fluor, Chlor, Brom, Cyano, Nitro, Hydroxycarbonyl, Methyl, Ethyl, n-oder i-Propyl, Methoxy, Ethoxy, Methoxycarbonyl, Ethoxycarbonyl oder Trifluormethyl,

sowie für den Fall, daß Ar für Pyridyl steht, auch deren Hydrochloridsalze.

4. Verfahren zur Herstellung von Pyranylalkyl-benzyl-ether der Formel (I),

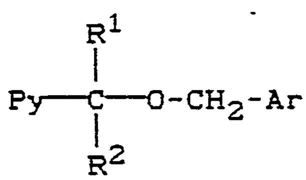

(I)

in welcher

$R^1$ und $R^2$ unabhängig voneinander jeweils für Wasserstoff oder Alkyl stehen,

Py für einen Rest

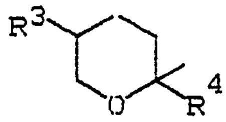

oder für einen Rest

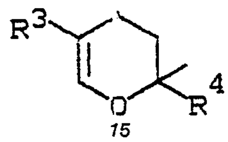

steht, wobei

$R^3$ für Wasserstoff oder Alkyl steht,

$R^4$ für Alkyl, Aralkyl, Alkoxyalkyl oder Aralkoxyalkyl steht und

Ar für jeweils gegebenenfalls substituiertes Phenyl, Naphthyl, Pyridyl oder Pyrimidyl steht,

dadurch gekennzeichnet, daß man Pyranylalkylcarbinole der Formel (II),

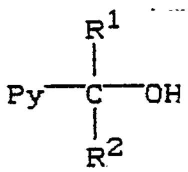

(II)

in welcher

$R^1$, $R^2$ und Py die oben angegebene Bedeutung haben,

mit Arylalkylhalogeniden der Formel (III),

Ar-$CH_2$-Hal (III)

in welcher

Ar die oben angegebene Bedeutung hat und

Hal für Halogen steht,

gegebenenfalls in Gegenwart eines Verdünnungsmittels und gegebenenfalls in Gegenwart eines Säurebindemittels umsetzt,

und daß man gegebenenfalls deren Salze erhält, wenn man Pyranylalkyl-benzyl-ether der Formel (I) mit Salzen umsetzt.

5. Herbizide Mittel, gekennzeichnet durch einen Gehalt an mindestens einem Pyranylalkyl-benzyl-ether der Formel (I) gemäß den Ansprüchen 1 bis 4.

6. Verfahren zur Bekämpfung von Unkraut, dadurch gekennzeichnet, daß man Pyranylalkyl-benzyl-ether der Formel (I) gemäß den Ansprüchen 1 bis 4 auf Unkräuter und/oder ihren Lebensraum einwirken läßt.

7. Verwendung von Pyranylalkyl-benzyl-ether der Formel (I) gemäß den Ansprüchen 1 bis 4 zur Bekämpfung von Unkräutern.

8. Verfahren zur Herstellung von herbiziden Mitteln, dadurch gekennzeichnet, daß man Pyranylalkyl-benzyl-ether der Formel (I) gemäß den Ansprüchen 1 bis 4 mit Streckmitteln und/oder oberflächenaktiven Mitteln vermischt.

9. Pyranylalkylcarbinole der Formel (IIa),

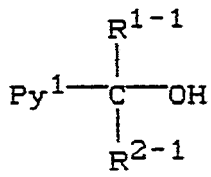

(IIa)

in welcher

$R^{1-1}$ und $R^{2-1}$ unabhängig voneinander jeweils für Wasserstoff oder Alkyl stehen,

$Py^1$ für einen Rest

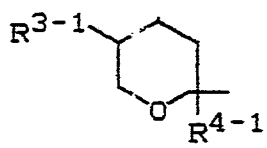

oder für einen Rest

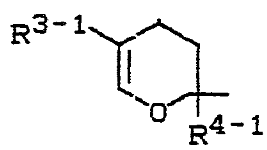

steht, wobei

$R^{3-1}$ für Wasserstoff oder Alkyl steht,

$R^{4-1}$ für Alkyl, Aralkyl, Alkoxyalkyl oder Aralkoxyalkyl steht,

ausgenommen sind jedoch die Verbindungen der Formeln

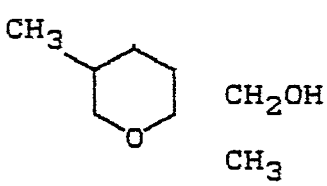

,

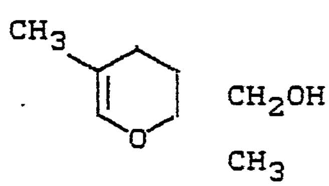

und

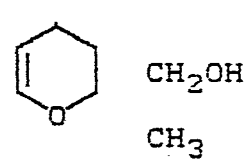

Europäisches Patentamt

# EUROPÄISCHER RECHERCHENBERICHT

Nummer der Anmeldung: EP 86107472.2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | US - A - 3 124 599 (GUEST)<br>* Spalte 2, Zeilen 5-30 *<br>-- | 1,9 | C 07 D 309/06<br>C 07 D 309/24<br>C 07 D 405/12<br>A 01 N 43/16<br>A 01 N 43/40 |
| A | CHEMICAL ABSTRACTS, Band 86, Nr. 13, 28. März 1977, Columbus, Ohio, USA<br>KEIKO, N.A.; STEPANOVA, L.G.; KALIKHMAN, I.D.; VORONKOV, M.G. "Some chemical properties of 2-formyl-2,5-dialkoxy(alkylthio)-2,3-dihydro-γ-pyrans."<br>Seite 516, Spalte 2, Zusammenfassung-Nr. 89527y<br>& Khim. Geterotsikl. Soedin. 1976, (10), 1319-22<br>-- | 1 | |
| A | CHEMICAL ABSTRACTS, Band 91, Nr. 25, 17. Dezember 1979, Columbus, Ohio, USA<br>CARDELLINA, JOHN H., II; MOORE, RICHARD E.; ARNOLD, EDWARD V.; CLARDY, JON. "Structure and absolute configuration of malyngolide, an antibiotic from the marine blue-green alga Lyngbya majuscula Gomont."<br>Seite 661, Spalte 2, Zusammenfassung-Nr. 211 192f<br>& J.Org.Chem. 1979, 44(23), 4039-42<br>-- | 9 | RECHERCHIERTE SACHGEBIETE (Int. Cl.4)<br>C 07 D 309/00<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 08-09-1986 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 86107472.2

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | CHEMICAL ABSTRACTS, Band 88, Nr. 23, 5. Juni 1978, Columbus, Ohio, USA<br>JENNER, GERARD; ABDI-OSKOUI, HOSSEIN; RIMMELIN, JEAN. "Pericyclic reactions under pressure. 6. Effect of high pressure in heterodiene syntheses between unsaturated carbonyl derivatives and acrylic and methacrylic dienophiles." Seite 568, Spalte 1, Zusammenfassung-Nr. 169885v<br>& Bull. Soc. Chim. Fr. 1977, (9-10, Pt.2), 983-7<br>---- | 1,9 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 08-09-1986 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument